# EUROPEAN PATENT SPECIFICATION

(11) **EP 3 541 433 B1**
(45) Date of publication and mention of the grant of the patent: **30.09.2020**
(21) Application number: 17787628.1
(22) Date of filing: 25.09.2017
(51) Int. Cl.: A61L 2/20, B66B 31/02, A61L 101/10

(54) **ESCALATOR HANDRAIL BELT DECONTAMINATION SYSTEM AND METHOD**
SYSTEM UND VERFAHREN ZUR DEKONTAMINATION DES HANDLAUFS VON ROLLTREPPEN
SYSTÈME ET MÉTHODE DE DÉCONTAMINATION DE COURROIE DE MAIN COURANTE D'ESCALIER MÉCANIQUE

(30) Priority: 18.11.2016 LT 2016112
(43) Date of publication of application: 25.09.2019
(73) Proprietor: UAB "Airplus1 Lituanica", 95112 Klaipeda (LT)
(72) Inventor: CAJAUSKIS, Saulius, LT-92382 Kunkiai (LT); J. MARKEVICIUS, Paul, London W3 7TN (GB); BUNOKAS, Modestas, Klaipeda (LT)
(74) Representative: Klimaitiene, Otilija
(86) International application number: PCT/IB2017/055806
(87) International publication number: WO 2018/091985

(56) References cited:
- EP-A1- 0 431 648
- WO-A1-2013/042144
- JP-A- 2012 046 272
- JP-B2- 5 793 846

## Description

### Technical field of the invention

The invention relates to moving walkways handrail sanitizing system and method and in particular the invention relates to decontamination system for moving handrail belts of an escalator using ozone gas.

### Background of the invention

Escalators and moving walkways as a means of pedestrian conveyance are present in every modern city around the world, sited often in places where elevators would be impractical. Efficient and safe mobility transport devices are vital for the huge numbers of people travelling from home to a place of work or leisure who rely on the infrastructure as part of their daily lives. Principal areas of usage include transit systems, airports, shopping malls, department stores, convention centres, arenas, stadiums, hotels, hospitals, public and large commercial buildings - all require the use of escalators and moving walkways to manage large numbers and constant flows of people.

Otis, a leading manufacturer of escalators estimate that in nine days the equivalent of the entire world's population travel on their lifts, escalators and moving walkways. National Elevator Industry Inc. in the USA estimate that 35,000 escalators in use will transport 105 billion passengers per year in the USA alone. In the European Union there are an estimated 75,000 escalators in use, with the London Underground System, the third busiest metro in Europe, carrying 1.265 billion passengers in 2013/14. Many more escalators are being added with the Crossrail link infrastructure, set to carry an additional 1.5million new passengers daily across greater London.

New developments in handrail technology using thermoplastic urethane, have proven they are more durable than traditional rubber handrails, easier to install and maintain with a longer operational life and are 'ozone friendly.' Handrails have always served as an important safety feature as a handgrip, incorporating motion indicator dots, spaced evenly on the handrail providing cues for speed, direction, distance and motion and now include handrail advertising graphics. Both features have been proven to offer added safety benefits of fewer trips and falls and for a more natural step on and off the escalator. The advent of more handrail advertising globally will assist as a visual stimulus to encourage users to use the handrails more. Observations made on passenger use of escalators will quickly reveal the importance of the handrail for safety, with the closest hand instinctively reaching the handrail at the point of boarding while taking the first step onto the moving escalator. Further observations will reveal a high percentage of passengers maintaining hand contact throughout the escalator ride, as habituated normal behaviour for safety, balance and comfort every time they use an escalator. Increased awareness of the handrail and the potential for maintaining and increasing the use of the handrails, coincides with another human propagated behaviourism: the proliferation and transmission of bacteria and viruses, carried unknowingly on the hands of a constant flow of millions of people, especially during rush hour peak travel periods. Oxford Circus station in the London Underground network has 98 million users per year alone. Many of the diseases that people suffer from, including norovirus, flu and a number of new forms of infectious and antibiotic-resistant pathogens, including the threat of international disease transmission, can be spread by such contact.

The potential for infection transmission is high by virtue of the behaviour patterns of escalator users, exposure to the bacteria on the handrails and the sheer numbers of people using handrails on escalators daily. There is a vital need to introduce an effective system for disinfecting the handrails, to keep the microorganisms at bay and reduce the risk of infection transmission. Existing methods of cleaning handrails, using detergents or similar chemicals with a wiping operation does not fully disinfect the handrails and remove the bacteria being constantly added to from continuous use. The sanitation materials, process and frequency of cleaning is inadequate and simply unable to effectively reduce or kill bacteria on handrails. Cleaning practices and procedures of handrails will vary owing partly to cost reasons or lack of perceived need for more effective decontamination, from occasional cleaning or largely ignored until the handrails are replaced or cleaned during maintenance.

The impact of infection from harmful bacteria for humans is significant as a serious health hazard. There is a high correlation between bacterial infection and pneumonia for example, for certain people more susceptible to infection requiring treatment in hospitals. Many infections transmitted by a lack of effective decontamination in this way place a significant financial cost burden on our health services globally. In addition to the loss of life from preventable infections, there are huge equally preventable hospitalization costs. Each winter and often throughout the year, the total number of work days lost through sickness (winter flu epidemics for example) runs into the billions.

International patent application, publication No. WO2013/042144, discloses an apparatus for cleaning flooring surfaces. The apparatus is provided with an ozone generating unit (16) and insertion means (17) of ozone into the liquid. The insertion means are connected to the ozone generating unit (16) and to the liquid dispenser means (5) in order to insert the ozone produced by the generating unit into the liquid at the dispenser means. The apparatus is therefore able to generate ozone, insert it into a cleaning liquid, such as for example simply water, dispense the liquid thus added to and recuperate the liquid together with any dirt from the surface to be cleaned on which the ozone exerts its action. The drawback of the apparatus is that it is not suitable for use with elevator handrails and does not have a secondary ozone detection and destruction means.

Japan patent application, publication No. JP2012046272, discloses a handrail sterilizing apparatus for a man conveyor, which sterilizes a moving handrail by using ozone. The handrail sterilizing apparatus includes a treatment device in which a treatment chamber for sterilizing the moving handrail is formed; an ozonizer for supplying the ozone into the treatment chamber; an air exhaust pump for keeping the treatment chamber depressurized by discharging air in the treatment chamber to the outside; and an ozone decomposition catalyst for decomposing the ozone in the air exhausted by the air exhaust pump. Additionally, in the treatment device, through-holes and opened in the treatment chamber are formed. The moving handrail 4 is disposed so as to pass through the through-hole, the treatment chamber, and the through-hole in order. Main disadvantage of the disclosed system is that it does not have a secondary ozone detection and destruction means.

Japan patent No. JP5793846 discloses a control device of a man conveyor which includes a disinfection device disinfecting a moving handrail by using the ozone, and which is appropriately controlled considering the influence of ozone on passengers.The man conveyor includes a disinfection device disinfecting the moving handrail by using ozone; and an activation means for activating the man conveyor by a predetermined operation. When the predetermined operation is performed for the activation means, the disinfection device 10 is moved to perform a predetermined disinfection operation for disinfecting the moving handrail, and then the man conveyor is changed to normal operation. Main disadvantage of the disclosed system is that it does not have a secondary ozone detection and destruction means. European patent application, publication No. EP0431648, discloses a process that deals with the sick building syndrome and building related illnesses, and solves volatile organic compound and biological pollution problems associated with large confined areas through which forced air is circulated, and the area is utilized for sociatary functions or activities, e.g., habitation by animals and/or equipment or other materials sensitive to pollutants or contaminants utilized in a societary activity within such areas, which comprises treating the circulating air in an air treatment zone with ozone in a depollution catalytic zone removed from contact with humans and/or the sensitive equipment to remove pollutants and contaminants from the air, and thereafter, the treated air is treated to remove ozone before being returned to the cycle flow within the confined area located outside of the air treatment zone. The drawback of the apparatus is that it is not suitable for use with elevator handrails.

Closest prior art is disclosed in patent application No. CN2009249568U describing an automatic disinfection device for elevator handrail comprising an ultraviolet pipe, an ozoniser and a disinfectant box, wherein the disinfectant box is arranged on one side of the elevator handrail, a fan is mounted below or above the elevator handrail, the ultraviolet pipe and a box body are mounted in sequence, and the ozoniser is mounted in the box. The disclosed system has at least two drawbacks: firstly, the system does not provide any means for controlling concentration of ozone in the vicinity of disinfection area and further from it and secondly it does not remove ozone from the handrail belt before interaction with escalator users. These drawbacks may lead to health risks for users related to harmful concentration of ozone gas in the area not intended for disinfection.

The shortcomings of the prior art as described are addressed directly by the installation of an ozone technology solution.

### Short description of the invention

The escalator handrail decontamination system (EHDS) comprises at least one sealed decontamination unit (1', 1"), for safe and localized decontamination environment, comprising at least one ozone generator, at least one ozone concentration sensor (3) at the decontamination zone, at least one ozone destruction unit (4) for converting ozone to oxygen; at least one ozone safety sensor (5', 5") for monitoring ozone level in the area surrounding the moving handrail belt (6); at least one secondary ozone destruction unit (7', 7") downstream of the at least one safety sensor (5', 5"); an intermediate controller (8) and a central controller (9). The ozone output from the EHDS ozone generator is constantly monitored by an ozone concentration sensor (3) to ensure minimum efficacy levels of ozone are maintained and to ensure ozone output remains within designated safety levels. The EHDS is positioned at the unseen lower section of the revolving handrail.

### Brief description of the drawings

The function and efficacy of the EHDS is outlined in the drawings and descriptions. The individual parts in the drawing are not to scale, but intended to illustrate the application and principles of the invention.

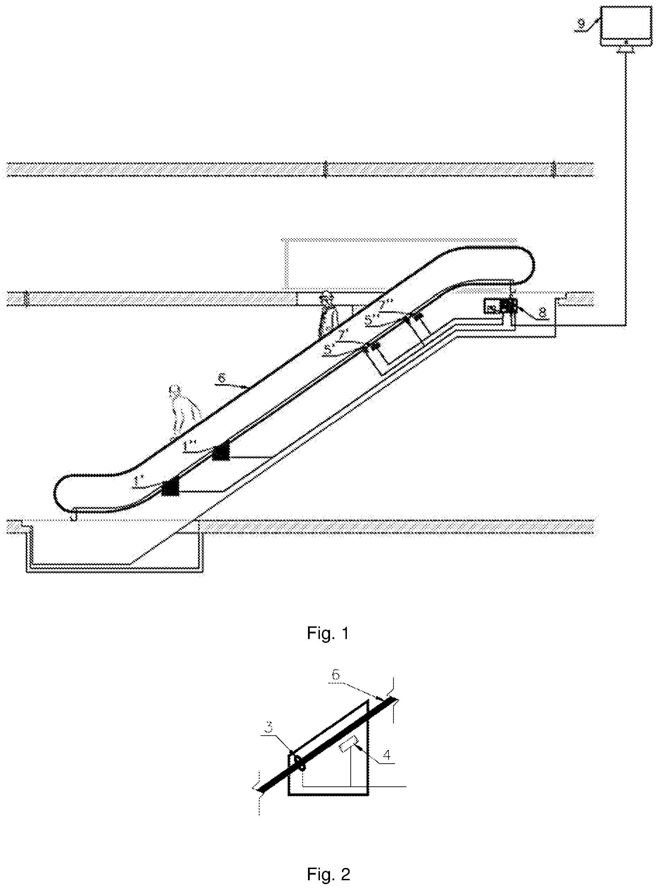

- Fig. 1: shows a perspective view of the EHDS installed within an escalator environment;
- Fig. 2: shows the decontamination unit of EHDS.

### Detailed description of the invention

The EHDS comprises at least one sealed decontamination unit (1', 1") comprising an ambient air assisted ozone generator (not shown in the drawings), wherein the ozone generator may be inside the decontamination unit (1) or outside (not shown in the drawings), positioned close to a moving handrail belt (6); an ozone sensor (3) to monitor ozone concentration close to and near the surface of the moving handrail belt (6); a first ozone destruction unit (4) for removing ozone from the moving handrail belt surface. In addition the system comprises at least one ozone safety sensor (5', 5") for monitoring ozone presence on the moving handrail belt (6) after the moving handrail belt leaves the decontamination unit or units (1', 1") and at least one further destruction unit (7', 7") positioned after the at least one ozone safety sensor (5', 5") or each ozone safety sensors (5', 5") in case of multiple sensors (5', 5"). Also an intermediate controller (8) is installed at the site for communication of all system elements with the central control unit (9).

The handrail belt (6), moving at approximately 1-2 feet (0.30-0.61) per second, enters the at least one decontamination unit (1', 1"), which is secured to the escalator truss frame, through entrance cavity which has a built-in sealed design preventing the generated ozone from leaving the at least one decontamination unit (1', 1")) through said entrance cavity. The ozone is generated and transferred locally to the zone of disinfection and preferably at close proximity to the moving handrail belt (6) surface. The ozone concentration is monitored by a concentration sensor (3) at the zone of disinfection. The sensor is positioned so that it can sense the ozone concentration level as close to the surface of the moving handrail belt (6) as possible. The first ozone destruction unit (4), which may be a heating unit for heating the handrail belt and its close surroundings, removes ozone at least from the surface of the handrail belt (6) before it leaves the at least one decontamination unit (1', 1") through exit cavity which has a built-in sealed design preventing the generated ozone from leaving the decontamination unit (1', 1") through said exit cavity. The ozone destruction unit (4) removes ozone by heating the ozone gas and converting it to oxygen.

The moving handrail belt may enter several subsequent decontamination units (1', 1") for maximum efficacy.

After the handrail belt leaves the at least one decontamination unit (1', 1") or, if several are used, the last decontamination unit, it passes through at least one ozone safety sensor (5', 5") monitoring the presence of ozone on the surface of the handrail belt. If ozone is detected, this area of the handrail belt and the area adjacent to it is treated by a secondary ozone destruction unit (7', 7"), such as a heating unit. Any number of ozone safety sensors with secondary destruction units downstream of the at last one decontamination unit may be used depending on the escalator construction. In this way the handrail belt is efficiently disinfected by ozone which is removed from the surface of the handrail belt before it emerges for contact with users. If the EHDS system comprises more than one decontamination unit (1', 1"), the ozone treatment in each of them is controlled in such a way to ensure efficacy and safety. If the EHDS system comprises more than one safety sensor (5', 5"), the secondary ozone destruction unit (7', 7"), or units (7', 7"), after each of the safety sensor (5', 5"), are operated so that ozone is removed from the surface of the handrail belt before it emerges for contact with users.

According to each escalator site, installation of the EHDS may include multiples of these key parts, positioned approximately one meter apart, according to maximum decontamination impact, determined for example by longer escalators or moving walkways with correspondingly longer handrails, with more contact areas to be covered by the EHDS for efficacy.

Although numerous characteristics and advantages together with structural details and features have been listed in the present description of the invention, the description is provided as an example fulfilment of the invention. Without departing from the principles of the invention, there may be changes in the details, especially in the form, size and layout, in accordance with most widely understood meanings of the concepts and definitions used in claims.

## Claims

1. Use of a decontamination system for disinfecting escalator hand-rails, wherein the decontamination system comprises at least one sealed decontamination unit (1', 1") comprising at least one ozone generator, at least one ozone concentration sensor (3), at least one first 5-ozone destruction unit (4); and further comprises at least one ozone safety sensor (5', 5"), positioned after the at least one decontamination unit (1', 1"), at least one secondary ozone destruction unit (7', 7"), positioned after the at least one safety sensor (5', 5"), an intermediate controller (8), and a central controller (9).

2. Method for decontamination of escalator handrail belt (6) using the decontamination system as defined in claim 1 **characterised in that** ozone level is monitored in the at least one sealed escalator handrail belt (6) decontamination unit (1', 1"), ozone is converted into oxygen by the at least one first ozone destruction unit (4) in the at least one sealed decontamination unit (1', 1"), ozone level is further monitored downstream of the at least one sealed decontamination unit (1', 1") by the at least one ozone safety sensor (5', 5"), moving handrail belt (6) is further treated with at least one secondary ozone destruction unit (7', 7").

## Patentansprüche

1. Verwendung eines Dekontaminationssystems zum Desinfizieren von Rolltreppen-Handläufen, wobei das Dekontaminationssystem mindestens eine abgedichtete Dekontaminationseinheit (1', 1") umfasst, umfassend mindestens einen Ozongenerator, mindestens einen Ozonkonzentrationssensor (3), mindestens eine erste Ozonzerstörungseinheit (4); und weiter mindestens einen hinter der mindestens einen Dekontaminationseinheit (1', 1") positionierten Ozonsicherheitssensor (5', 5"), mindestens eine hinter dem mindestens einen Ozonsicherheitssensor (5', 5") positionierte sekundäre Ozonzerstörungseinheit (7', 7"), eine zwischenliegende Steuerung (8) und eine zentrale Steuerung (9).

2. Verfahren zur Dekontamination von einem Rolltreppen-Handlaufband (6) unter Verwendung des Dekontaminationssystem nach Anspruch 1, **dadurch gekennzeichnet, dass** das Ozonlevel in der mindestens einen abgedichteten Rolltreppen-Handlaufband (6)-Dekontaminationseinheit (1', 1") überwacht wird, das Ozon in der mindestens einen abgedichteten Dekontaminationseinheit (1', 1") von der mindestens einen Ozonzerstörungseinheit (4) zu Sauerstoff umgewandelt wird, das Ozonlevel weiter der mindestens einen abgedichteten Dekontaminationseinheit (1', 1") nachgelagert durch den mindestens einen Ozonsicherheitssensor (5', 5") überwacht wird, ein sich bewegendes Handlaufband (6) weiter mit mindestens einer sekundären Ozonzerstörungseinheit (7', 7") behandelt wird.

## Revendications

1. Utilisation d'un système de décontamination pour désinfecter des mains courantes d'escalier mécanique, dans laquelle le système de décontamination comprend au moins une unité de décontamination scellée (1', 1") comprenant au moins un générateur d'ozone, au moins un capteur de concentration d'ozone (3), au moins une première unité de destruction d'ozone (4) ; et comprend en outre au moins un capteur de sécurité de l'ozone (5', 5") positionné après la au moins une unité de décontamination (1', 1"), au moins une unité secondaire de destruction d'ozone (7', 7"), positionnée après le au moins un capteur de sécurité (5', 5"), un dispositif de commande intermédiaire (8) et un dispositif de commande central (9).

2. Procédé de décontamination d'une courroie de main courante d'escalier mécanique (6) à l'aide du système de décontamination tel que défini dans la revendication 1, **caractérisé en ce que** le niveau d'ozone est surveillé dans la au moins une unité de décontamination (1', 1") de courroie de main courante d'escalier mécanique (6), l'ozone est converti en oxygène par la ou au moins une première unité de destruction d'ozone (4) dans la au moins une unité de décontamination scellée (1', 1"), le niveau d'ozone est en outre surveillé en aval de la au moins une unité de décontamination scellée (1', 1") par le au moins un capteur de sécurité de l'ozone (5', 5"), un mouvement de la courroie de main courante (6) est en outre traité avec au moins une unité secondaire de destruction d'ozone (7', 7").
